# EUROPÄISCHE PATENTSCHRIFT

(11) **EP 0 388 868 B1**
(45) Veröffentlichungstag und Bekanntmachung des Hinweises auf die Patenterteilung: **10.11.1993**
(21) Anmeldenummer: 90105192.0
(22) Anmeldetag: 20.03.1990
(51) Int. Cl.: C07C 31/20, C07C 29/14, C07D 307/20

(54) **Verfahren zur Herstellung von Butandiol-1,4 und Tetrahydrofuran**
Process for the production of 1,4-butane diol and tetrahydrofuran
Procédé de préparation du butanediol-1,4 et du tétrahydofurane

(30) Priorität: 22.03.1989 DE 3909485
(43) Veröffentlichungstag der Anmeldung: 26.09.1990
(73) Patentinhaber: BASF Aktiengesellschaft, 67063 Ludwigshafen (DE)
(72) Erfinder: Fischer, Rolf, Dr., D-6900 Heidelberg (DE); Frank, Juergen, Dr., D-6830 Schwetzingen (DE); Merger, Franz, Dr., D-6710 Frankenthal (DE); Weyer, Hans-Juergen, Dr., D-6800 Mannheim 1 (DE)

(56) Entgegenhaltungen:
- US-A- 4 064 145
- US-A- 4 161 616

## Beschreibung

Diese Erfindung betrifft ein Verfahren zur Herstellung von Butandiol-1,4 und Tetrahydrofuran, bei dem man 4-Hydroxybutyraldehyd in ein Gemisch der Acetale 2-(4-Oxobutoxy)tetrahydrofuran und 2,2-Oxybistetrahydrofuran überführt, aus dem man durch Hydrierung Butandiol-1,4 und Tetrahydrofuran gewinnt.

Es ist bekannt, daß man 4-Hydroxybutyraldehyd durch katalytische Hydrierung in Butandiol-1,4 überführen kann, welches sich in Gegenwart von sauren Katalysatoren zu Tetrahydrofuran cyclisieren läßt.

Es wurde nun gefunden, daß man Gemische aus Butandiol-1,4 und Tetrahydrofuran besonders vorteilhaft herstellen kann, wenn man
a) 4-Hydroxybutyraldehyd der Formel 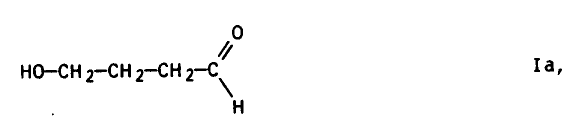 gegebenenfalls in Gegenwart von Butandiol-1,4, auf Temperaturen von 20 bis 300°C erhitzt, und
b) die dabei gebildeten Acetale der Formeln 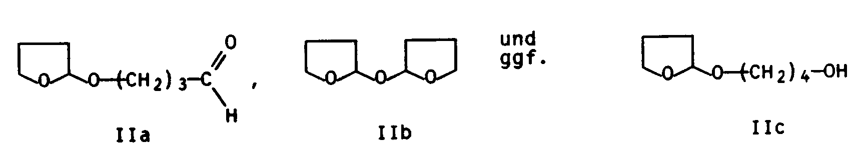 bei Temperaturen von 50 bis 300°C und Drücken von 1 bis 350 bar katalytisch hydriert.

über die Acetale der Formeln IIa und IIb war bekannt, daß sie als Nebenprodukte bei der Umsetzung von Allylalkohol mit Kohlenmonoxid und Wasserstoff in Gegenwart von durch Phosphine modifizierten Rhodiumkatalysatoren entstehen. Die hierbei erhaltenen Produktgemische enthalten neben dem gewünschten 4-Hydroxybutyraldehyd 2-Methyl-3-hydroxypropionaldehyd, Butyrolacton, 2-Methyl-3-(tetrahydro-2-furanyloxy)-propanol auch die Acetale der Formeln IIa und IIb (s. Chemiker-Zeitung 101 (1977), Seiten 343 bis 350).

über die Hydrierung des Acetals der Formel IIa war bekannt, daß sich 97,5 %iges 1,4-Butandiol mit einem Gehalt von 0,3 % eines Gemisches aus der Verbindung IIa, 1,4-Bis(tetrahydro-2-furyloxy)butan und Hochsiedern bei 100°C durch Hydrierung in Gegenwart eines Pd-Katalysators reinigen läßt (Jpn. Kokai 61 197 534; C.A. 106, 49.607). Dabei geht der Gehalt des Gemisches an der Verbindung IIa, 1,4-Bis(tetrahydro-2-furyloxy)butan und Hochsiedern auf 0,24 % zurück, so daß offenbar nur 0,06 % umgesetzt werden. Welche Verbindungen bei der Hydrierung entstehen, wird nicht angegeben.

Nach dem neuen Verfahren dieser Erfindung erhitzt man 4-Hydroxybutyraldehyd, der im Gleichgewicht mit seinem cyclischen Halbacetal der Formel 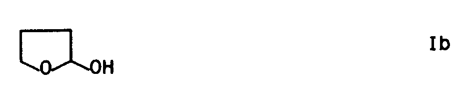
steht, auf Temperaturen von 20 bis 300°C, insbesondere 80 bis 150°C. Die Umsetzung kann diskontinuierlich oder kontinuierlich, drucklos oder unter erhöhtem oder vermindertem Druck durchgeführt werden. Um eine Oxidation der Aldehydgruppe im 4-Hydroxybutyraldehyd zu unterbinden, kann es vorteilhaft sein, die Umsetzung in Gegenwart eines unter den Reaktionsbedingungen inerten Gases, wie Z.B. Stickstoff, Argon oder Kohlendioxid, durchzuführen. Die Reaktionszeiten betragen für die diskontinuierliche Umsetzung 0,5 bis 6 Stunden, insbesondere 1 bis 4 Stunden.

Nach einer Variante des Verfahrens dieser Erfindung wird 4-Hydroxybutyraldehyd in Gegenwart von Butandiol-1,4 erhitzt, wobei sich das Acetal der Formel IIc bildet, das bei der erfindungsgemäßen Hydrierung ebenfalls in ein Gemisch aus Tetrahydrofuran und Butandiol-1,4 übergeht.

Die Bildung der Acetale der Formeln IIa, IIb und IIc läßt sich durch die folgenden Formeln verdeutlichen: 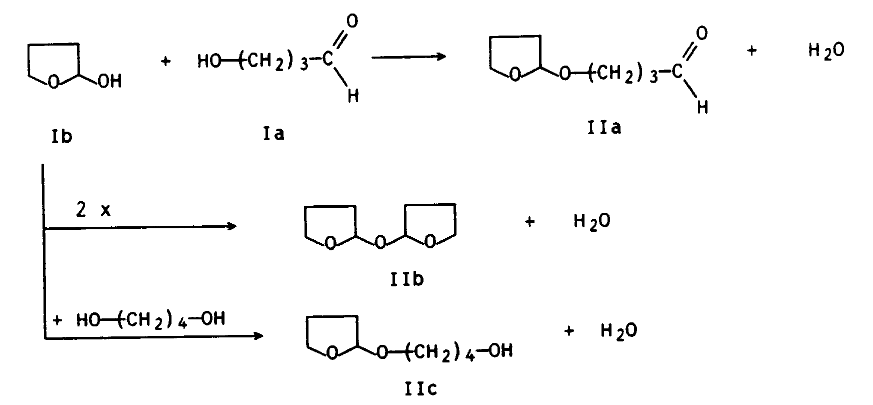
Das Acetal der Formel IIc wird unter den gleichen Bedingungen wie die Acetale IIa und IIb hergestellt. Bei dieser Verfahrensvariante ist es jedoch empfehlenswert, einen überschuß an Butandiol-1,4 gegenüber 4-Hydroxybutyraldehyd anzuwenden. So gibt man zu einem Mol der Verbindungen Ia + Ib zweckmäßigerweise 1 bis 5, insbesondere 1,5 bis 3 Mol Butandiol-1,4.

Die Umsetzung gemäß Stufe (a) kann in Gegenwart von Lösungsmitteln durchgeführt werden, die unter den Reaktionsbedingungen inert sind. Hierfür geeignet sind beispielsweise Wasser, aliphatische, cycloaliphatische oder aromatische Kohlenwasserstoffe, wie n-Hexan, Cyclohexan, Methylcyclohexan, Benzol, Toluol, oder Chlorkohlenwasserstoffe, wie Methylenchlorid, Chloroform, Tetrachlormethan, 1,2-Dichlorethan, oder Ether, wie Methyl-tert.-butylether, Dioxan, Tetrahydrofuran oder Gemische dieser Lösungsmittel.

Falls Lösungsmittel mitverwendet werden, setzt man die Ausgangsverbindung der Formeln Ia/Ib als 1 bis 90 gew.-%ige Lösung, insbesondere als 5 bis 20 gew.-%ige Lösung, ein.

Ein besonderer Vorteil des erfindungsgemäßen Verfahrens besteht darin, daß die Herstellung der Acetale IIa, IIb und IIc eine Mitverwendung von Katalysatoren nicht erfordert.

Um die Bildung der Acetale IIa und IIb zu beschleunigen, kann es aber in manchen Fällen vorteilhaft sein, saure Mittel zuzusetzen. Geeignete saure Mittel sind beispielsweise Sulfonsäuren, Lewissäuren, nicht oxidierende Mineralsäuren, niedere Fettsäuren oder saure Kationenaustauscher. Beispiele hierfür sind Mineralsäuren, wie Schwefelsäure, Chlorwasserstoffsäure, Bromwasserstoffsäure, Sulfonsäuren, wie p-Toluolsulfonsäüre oder Lewissäuren, wie Bortrifluorid oder Zinkchlorid, ferner niedere aliphatische Carbonsäuren, wie Ameisensäure, Essigsäure oder Propionsäure sowie Zeolithe oder saure Kationenaustauscher. Geeignete saure Kationenaustauscher sind beispielsweise solche, die aus vernetztem Polystyrol mit Sulfonsäuregrupppen oder Phenolharzen mit Sulfonsäuregruppen aufgebaut sind.

Die genannten Säuren werden zweckmäßigerweise in katalytischen Mengen, z.B. von 0,001 bis 0,25 Mol je Mol Ia/Ib eingesetzt. Besonders bevorzugt werden stark saure Kationenaustauscher.

Die bei der Stufe (a) gebildeten Reaktionsgemische, die die Acetale der Formeln IIa und IIb bzw. IIc enthalten, können direkt, d.h. ohne vorherige Abtrennung von unumgesetztem 4-Hydroxybutyraldehyd für die nachfolgende Hydrierung eingesetzt werden. Verfährt man so, so kann es für die Hydrierung je nach Wahl des Katalysators vorteilhaft sein, das gebildete Wasser während oder nach Bildung der Acetale IIa und IIb bzw. IIc abzutrennen. Da sowohl das Acetal IIa als auch das der Formel IIb zu Tetrahydrofuran und Butandiol-1,4 hydrierbar sind, ist eine Trennung dieser Verbindungen vor der Hydrierung nicht notwendig.

Man kann die Acetale der Formeln IIa, IIb und IIc aber auch vor der Hydrierung aus den Reaktionsgemischen, die man bei der Stufe (a) erhält, isolieren. So kann man die gebildeten Acetale, gegebenenfalls nach Entfernung der genannten sauren Mittel, die man auf an sich bekannte Weise z.B. durch Neutralisation, Destillation oder Filtration vornimmt, durch Destillation von der nicht umgesetzten Ausgangsverbindung (Ia/Ib) und dem gebildeten Wasser abtrennen. Zur Aufarbeitung des Acetals der Formel IIc kann das im Reaktionsgemisch enthaltene Butandiol-1,4 ganz oder teilweise abgetrennt und gegebenenfalls in die Synthese zurückgeführt werden.

Um einen möglichst hohen Umsatz an dem Einsatzstoff Ia/Ib zu erzielen, kann es vorteilhaft sein, das bei der Umsetzung entstehende Wasser kontinuierlich aus dem Reaktionsgemisch abzudestillieren. Hat man Lösungsmittel, die mit Wasser Azeotrope bilden, eingesetzt, so können diese zusammen mit dem Wasser aus dem Reaktionsgemisch abdestilliert werden.

Die erfindungsgemäße Hydrierung der Acetale der Formeln IIa, IIb und IIc läßt sich durch das folgende Schema verdeutlichen: 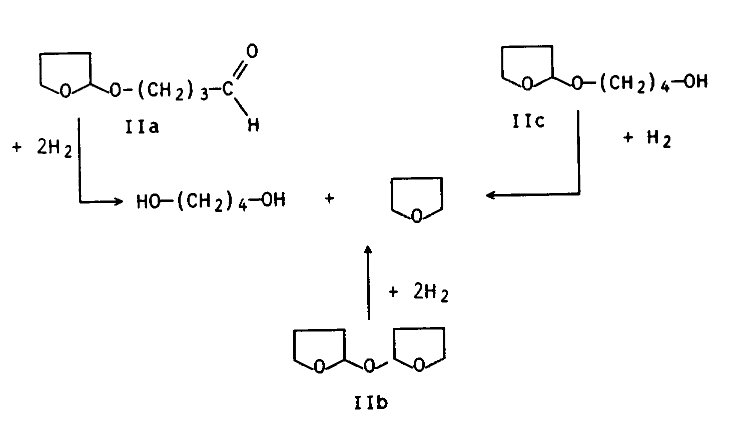
Neben Butandiol-1,4 und Tetrahydrofuran entsteht in untergeordnetem Maße 4,4'-Dihydroxydibutylether HO-(CH₂)₄-O-(CH₂)₄-OH. Dieses Etherdiol ist für die Herstellung von Polyestern geeignet und läßt sich wegen der bevorzugten Bildung von Tetrahydrofuran nicht durch Kondensation zweier Moleküle Butandiol-1,4 herstellen.

Man hydriert bei Temperaturen von 50 bis 300°C, insbesondere 100 bis 260°C und bei Drücken von 1 bis 350 bar, insbesondere 100 bis 300 bar. Die Hydrierung wird in der Gas- oder Flüssigphase durchgeführt. Man arbeitet diskontinuierlich, insbesondere aber kontinuierlich, in der Sumpf- oder Rieselfahrweise, wobei der Katalysator fest angeordnet ist. Es ist auch möglich, suspendierte Katalysatoren einzusetzen. Dabei haben sich Katalysatorbelastungen von 0,05 bis 1, insbesondere 0,1 bis 0,5 kg IIa + IIb je Liter Katalysator und Stunde, bewährt. Als Reaktoren können z.B. Rohrreaktoren oder Rohrbündelreaktoren verwendet werden. Als Katalysatoren kann man an sich bekannte Hydrierkatalysatoren einsetzen.

Bevorzugte Hydrierkatalysatoren sind solche, die Kupfer und/oder Metalle der VIII. Nebengruppe des Periodischen Systems der Elemente enthalten, insbesondere Nickel, Cobalt, Palladium, Platin, Rhodium und/oder Ruthenium. Die Katalysatoren werden als Trägerkatalysatoren oder als Vollkatalysatoren eingesetzt. Trägerkatalysatoren werden auf an sich bekannte Weise hergestellt, zweckmäßigerweise durch ein- oder mehrmaliges Imprägnieren des Trägermaterials mit einer wäßrigen Lösung der Metallsalze, Trocknen und Erhitzen der katalytischen Masse, wobei die Metallsalze in die Metalloxide übergehen. Vor ihrem Einsatz werden die Katalysatoren mit Wasserstoff behandelt, wobei die Oxide größtenteils zu den Metallen reduziert werden. Als Katalysatorträger sind z.B. Siliciumdioxid, Aluminiumoxid, Titandioxid, Aktivkohle, Silikate oder Zeolithe geeignet. Wenn nötig, können bei der Katalysatorherstellung Bindemittel, z.B. Graphit, verwendet werden.

4-Hydroxybutyraldehyd ist beispielsweise durch Hydroformylierung von Allylalkohol in Gegenwart von Rhodium-Komplexverbindungen, durch anodische Oxidation von Tetrahydrofuran (DE-OS 3 615 472) oder durch Isomerisierung von 2-Buten-1,4-diol mit Hilfe von Rhodiumverbindungen (DE-OS 3 718 897) zugänglich.

### Beispiele

### Beispiel 1

### a) Herstellung der Acetale der Formeln IIa und IIb

40 g nach DE-OS 3 718 897 hergestellter 4-Hydroxybutyraldehyd wurde unter Stickstoff drei Stunden lang zum Sieden (124°C) erhitzt. Dann wurde das Reaktionsgemisch abgekühlt und durch GC-Analyse untersucht. Es bestand zu 24 % aus unumgesetztem 4-Hydroxybutyraldehyd, zu 11 % aus Acetal der Formel IIb und zu 64 % aus Acetal der Formel IIa (jeweils Mol-%, bezogen auf eingesetzten 4-Hydroxybutyraldehyd). Durch fraktionierende Destillation wurden 8,3 g 4-Hydroxybutyraldehyd (21 %) vom Siedepunkt 77°C/33 mbar und 25,3 g eines Gemisches der Acetale der Formeln IIa und IIb (71 %) vom Siedepunkt 98 bis 118°C/27 bis 33 mbar erhalten.

Diese Arbeitsweise wurde bei unterschiedlichen Reaktionstemperaturen durchgeführt. Die Reaktionsbedingungen und Ergebnisse sind der folgenden Tabelle zu entnehmen:

| Reakt.-Temp. (°C) | Reakt.-Zeit (h) | Mol-% 2) | | | Selektivität (%) |
|---|---|---|---|---|---|
| | | HBA 1) | II a | II b | |
| 80 | 6 | 59 | 21 | 17 | 93 |
| 100 | 6 | 36 | 42 | 21 | 98 |
| 120 | 6 | 26 | 52 | 21 | 99 |

| | | | | | |
|---|---|---|---|---|---|
| 1) HBA = 4-Hydroxybutyraldehyd | | | | | |
| 2) nach quantitativer GC-Analyse | | | | | |

### b) Herstellung des Acetals der Formel IIc

20 g nach DE-OS 3 718 897 hergestellter 4-Hydroxybutyraldehyd wurde mit 41,4 g Butandiol-1,4 drei Stunden lang unter Stickstoff auf 120°C erhitzt.

Durch gaschromatographische Analyse wurde festgestellt, daß das Reaktionsgemisch 44 % Acetal der Formel IIc, 41 % Butandiol-1,4 und 2 % unumgesetzten 4-Hydroxybutyraldehyd enthielt.

### c) Hydrierung

120 g eines entsprechend Absatz (a) erhaltenen Gemisches aus den Acetalen der Formeln IIa (85 %) und IIb (15 %) wurden in Gegenwart von 14 g eines mit Wasserstoff aktivierten Kupferkatalysators (55,6 % CuO, 43,6 % Al₂O₃) 10 Stunden lang bei 175°C und 200 bar hydriert. Durch Destillation des Reaktionsaustrages wurden 66,5 g Tetrahydrofuran, 46,5 g Butandiol-1,4 und 2,5 g 4,4'-Dihydroxidibutylether erhalten.

### Beispiel 2 (Vergleich)

Die Hydrierung von Beispiel 1, Absatz (c) wurde wiederholt, wobei jedoch anstelle des Ausgangsgemisches 120 g frisch destillierter 4-Hydroxybutyraldehyd verwendet wurden. Durch gaschromatographische Analyse wurden im Reaktionsaustrag 51,7 g Butandiol-1,4 und nur 1,3 g Tetrahydrofuran gefunden.

### Beispiel 3

120 g des Acetal-Gemisches, das in Beispiel 1, Absatz (c) eingesetzt wurde, wurde in Gegenwart von 11 g eines mit Wasserstoff aktivierten Kobaltkatalysators (63,4 % CoO, 18,1 % CuO, 6,8 % Mn3O4, 3,1 % MoO₃, 3,3 % P₂O₅, 0,2 % Na₂O) 15 Stunden bei 190°C und 200 bar hydriert. Die gaschromatographische Analyse ergab, daß bei vollständigem Umsatz 53,9 g Tetrahydrofuran, 62,3 g Butandiol-1,4 und 1,8 g 4,4'-Dihydroxydibutylether entstanden waren.

### Beispiel 4 (Vergleich)

Die Hydrierung von Beispiel 3 wurde wiederholt, wobei jedoch anstelle des Acetal-Gemisches 120 g frisch destillierter 4-Hydroxybutyraldehyd verwendet wurden. Durch gaschromatographische Analyse wurden im Reaktionsgemisch nur 15 g Butandiol-1,4 und 0,6 g Tetrahydrofuran gefunden.

### Beispiel 5

10 g eines entsprechend Beispiel 1, Absatz (b) erhaltenen Gemisches aus 2-(4-Hydroxybutoxy)-tetrahydrofuran der Formel IIc (83 %) und 1.4-Bis-(2-tetrahydrofuranyloxy)-butan (17 %) wurden in Gegenwart von 1 g eines Palladium-Katalysators (10 % Pd auf Aktivkohle) 10 Stunden lang bei 100°C und 150 bar hydriert. Der Autoklavenaustrag bestand laut gaschromatographischer Analyse aus 20,7 % Tetrahydrofuran, 38,4 % Butandiol-1,4, 29,6 % 4,4'-Dihydroxydibutylether und 7,4 % 5,10-Dioxatetradecan-1.14-diol.

## Patentansprüche

1. Verfahren zur Herstellung von Gemischen, die Butandiol-1,4 und Tetrahydrofuran enthalten, dadurch gekennzeichnet, daß man
a) 4-Hydroxybutyraldehyd der Formel 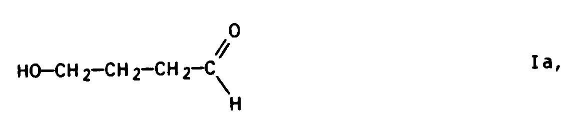 gegebenenfalls in Gegenwart von Butandiol-1,4 auf Temperaturen von 20 bis 300°C erhitzt, und
b) die dabei gebildeten Acetale der Formeln 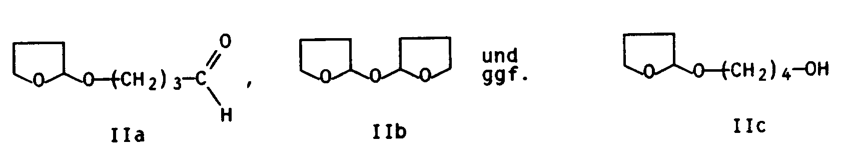 bei Temperaturen von 50 bis 300°C und Drücken von 1 bis 350 bar katalytisch hydriert.

2. Verfahren nach Anspruch 1, dadurch gekennzeichnet, daß man die Umsetzung nach Stufe (a) in Gegenwart eines unter den Reaktionsbedingungen inerten Lösungsmittels durchführt.

3. Verfahren nach den Ansprüchen 1 und 2, dadurch gekennzeichnet, daß man die Umsetzung nach Stufe (a) unter kontinuierlicher Abtrennung des Reaktionswassers durchführt.

4. Verfahren nach den Ansprüchen 1 bis 3, dadurch gekennzeichnet, daß man die Umsetzung nach Stufe (a) in Gegenwart von sauren Katalysatoren durchführt.

5. Verfahren nach Anspruch 1, dadurch gekennzeichnet, daß man die Hydrierung nach Stufe (b) in Gegenwart von Katalysatoren durchführt, die Kupfer und/oder Elemente der 8. Nebengruppe des Periodensystems der Elemente enthalten.

6. Verfahren nach den Ansprüchen 1 bis 5, dadurch gekennzeichnet, daß man die Hydrierung nach Stufe (b) ohne die vorherige Abtrennung von unumgesetztem 4-Hydroxybutyraldehyd durchführt.

## Claims

1. A process for preparing a mixture comprising 1,4-butanediol and tetrahydrofuran, which comprises
a) heating 4-hydroxybutyraldehyde, which has the formula 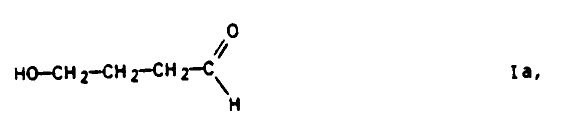 at 20-300°C in the presence or absence of 1,4-butanediol, and
b) subjecting the resulting acetals of the formulae 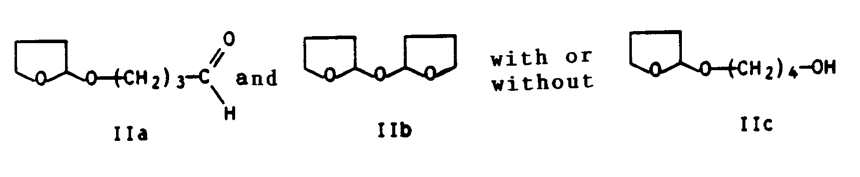 to a catalytic hydrogenation at 50-300°C and 1-350 bar.

2. A process as claimed in claim 1, wherein the reaction of stage (a) is carried out in the presence of a solvent which is inert under the reaction conditions.

3. A process as claimed in claim 1 or 2, wherein the reaction of stage (a) is carried out with continuous removal of the water of reaction.

4. A process as claimed in claims 1 to 3, wherein the reaction of stage (a) is carried out in the presence of an acidic catalyst.

5. A process as claimed in claim 1, wherein the hydrogenation of stage (b) is carried out in the presence of a catalyst comprising copper or an element of sub-group VIII of the periodic table of the elements.

6. A process as claimed in any of claims 1 to 5, wherein the hydrogenation of stage (b) is carried out without the prior removal of unconverted 4-hydroxybutyraldehyde.

## Revendications

1. Procède de préparation de mélanges qui contiennent du butanediol-1,4 et du tétrahydrofuranne, caractérisé en ce que,
a) on chauffe du 4-hydroxybutyraldéhyde de la formule 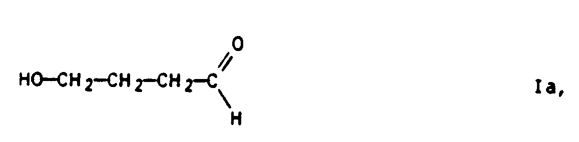 à des températures de 20 à 300°C, éventuellement en présence de butanediol-1,4 et
b) on hydrogène les acétals ainsi formés des formules 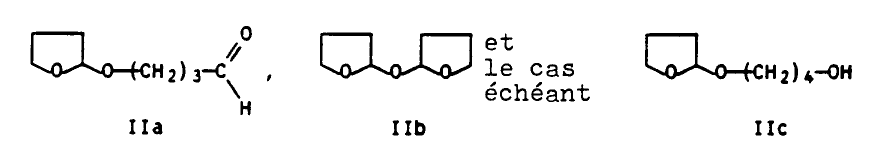 à des températures de 50 à 300°C et sous des pressions de 1 à 350 bars, par voie catalytique.

2. Procédé suivant la revendication 1, caractérisé en ce que l'on entreprend la réaction de l'étape a) en présence d'un solvant inerte dans les conditions réactionnelles.

3. Procédé suivant les revendications 1 et 2, caractérisé en ce que l'on entreprend la réaction de l'étape (a) sous séparation continue de l'eau de réaction.

4. Procédé suivant les revendications 1 à 3, caractérisé en ce que l'on entreprend la réaction de l'étape (a) en présence de catalyseurs acides.

5. Procédé suivant la revendication 1, caractérisé en ce que l'on entreprend l'hydrogénation de l'étape (b) en présence de catalyseurs qui contiennent du cuivre et/ou des éléments du huitième sous-groupe du système périodique.

6. Procédé suivant les revendications 1 à 5, caractérisé en ce que l'on entreprend l'hydrogénation de l'étape (b) sans la séparation préalable du 4-hydroxybutyraldéhyde non entré en réaction.
